# FASCICULE DE BREVET EUROPEEN

(11) **EP 0 531 498 B1**
(45) Date de publication et mention de la délivrance du brevet: **30.08.2006**
(21) Numéro de dépôt: 92908073.7
(22) Date de dépôt: 04.03.1992
(51) Int. Cl.: C12N 15/53, C12N 9/02, C12N 15/82, C12N 5/10, A01H 5/00, A01N 63/00

(54) **PRODUCTION DE PLANTES RESISTANTES AUX ATTAQUES DE SCLEROTINIA SCLEROTIORUM PAR INTRODUCTION D'UN GENE CODANT POUR UNE OXALATE OXYDASE**
HERSTELLUNG GEGEN SCLEROTINIA SCLEROTIORUM RESISTENTER PFLANZENDURCH EINFÜHRUNG EINES FÜR OXALATOXYDASE KODIERENDEN GENS
PRODUCTION OF PLANTS RESISTANT TO ATTACKS OF SCLEROTINIA SCLEROTIORUM BY INTRODUCING A GENE CODING FOR AN OXALATE OXIDASE

(30) Priorité: 05.03.1991 FR 9102874
(43) Date de publication de la demande: 17.03.1993
(73) Titulaire: Biogemma, 75001 Paris (FR)
(72) Inventeur: FREYSSINET, Georges, F-69450 St-Cyr-au-Mont-d'Or (FR); SAILLAND, Alain, F-69009 Lyon (FR)
(74) Mandataire: Flesselles, Bruno F.G.
(86) Numéro de dépôt international: PCT/FR1992/000195
(87) Numéro de publication internationale: WO 1992/015685

(56) Documents cités:
- WO-A-88/08450
- J. BIOL. CHEM. vol. 264, no. 9, 25 Mars 1989, pages 4896 - 4900; DRATEWKA-KOS E.,ET.AL.: 'Polypeptide structure of germin as deduced from cDNA sequencing'
- CHEMICAL ABSTRACTS, vol. 98, 1983, Columbus, Ohio, US; abstract no. 13533,
- AGRIC. BIOL. CHEM. vol. 52, no. 3, 1988, pages 743 - 748; KOYAMA H: 'Purification and characterization of oxaltae oxidase from Pseudomonas sp. OX-53'

## Description

La présente invention a pour objet l'utilisation d'un gène codant pour une oxalate oxydase, de la protéine codée par ce gène , des gènes chiméres comprenant ce gène pour la transformation de plantes en vue de leur conférer une résistance à des maladies fongiques.

La Sclerotiniose est une maladie fongique importante qui touche un grand nombre de dicotylédones. Le responsable, Sclerotinia sclerotiorum est un champignon polyphage qui présente peu de spécificité d'hôte.

Le champignon peut attaquer la plante soit directement au niveau de la tige, soit au niveau des feuilles puis gagner la tige, soit au niveau du capitule floral. Dans les deux premiers cas, la plante fane par rupture d'alimentation. Dans le dernier cas, la fleur fane compromettant la récolte.

Le champignon produit des enzymes lytiques qui dégradent la paroi cellulaire du végétal infecté et facilitent son développement dans la plante. Ces enzymes jouent un rôle important dans la pathogénicité, mais ne semblent pas suffisantes. Ce champignon produit aussi de l'acide oxalique (Godov et al., 1990). Cet acide oxalique provoque une baisse de pH dans les tissus infectés facilitant l'hydrolyse de la paroi cellulaire par les enzymes lytiques. Une réduction de la production d'acide oxalique ou une dégradation de cet acide oxalique doit permettre un ralentissement ou même une inhibition du développement du champignon.

Pour développer une plante résistante à "nos", on peut utiliser la stratégie de détoxication de l'acide oxalique. La dégradation de cet acide limitera les diminutions du pH intracellulaire du tissu végétal attaqué, les enzymes lytiques fonctionneront alors trop loin de leur pH optimum pour être réellement actives et efficaces. Il en résultera une baisse de la pathogénicité du champignon.

Pour atteindre cet objectif, on peut utiliser l'oxalate oxydase qui catalyse la réaction suivante :

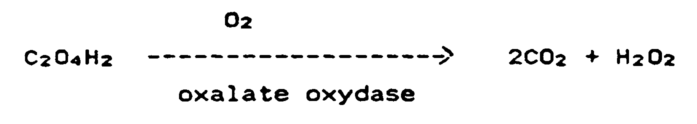

L'oxalate oxydase est isolée de plantes différentes, généralement des monocotylédones (Pieta et al., 1982): on peut, par exemple purifier la protéine d'orge en utilisant des techniques de chromatographie classique (gels de filtration Superdex G75 et d'échange d'ions MonoQ, Pharmacia), en suivant l'activité enzymatique selon le protocole colorimétrique suivant (Obzansky et Richardson, 1983) : 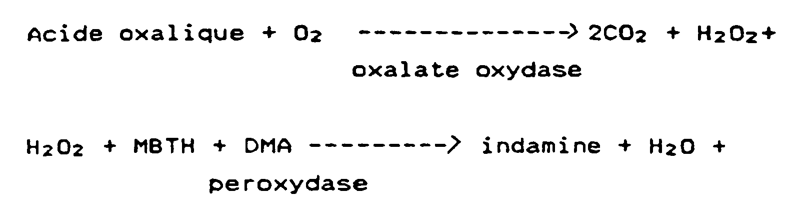 MBTH = 3-methyl-2-benzothiazolinone hydrazone
DMA = N,N,dimethylalinine

Ceci a permis de purifier une protéine qui, sur gel d'acrylamide en conditions dénaturantes, a une masse moléculaire de 26 000 daltons. Une partie de l'oxalate oxydase purifiée a été utilisée pour obtenir des anticorps antioxalate oxydase de lapin ; le reste de la protéine a été utilisé pour faire le séquençage de la protéine native (N-terminal) ou, après clivage au bromure de cyanogène, le séquençage de certains peptides internes. Les résultats obtenus sont les suivants :
N-terminal : TDPDPLQDF-VADLDGKAVSVNGHS
Peptide interne n° 2 : HFQFNVGKTEAY
L'ADNc

La comparaison des séquences peptidiques décrites ci-dessus avec les données contenues dans la banque protéique Swiss-Prot nous a permis d'identifier une protéine de blé appelée Germine et publiée en 1989 par Dratewka-kos et al. Des expériences ont été réalisées et nous ont permis de déterminer que l'ADNc publié par les auteurs code pour une protéine de 201 acides aminés et qui présente une activité oxalate oxydase. Pour la suite de la description des expériences présentées dans ce brevet nous reprendrons la numérotation des nucléotides de la figure 2 de l'article des auteurs publiés dans J. Biol. Chem., 264, 4896-4900.

La séquence de cet ADNc a une longueur de 1 075 nucléotides avec un 5' non traduit de 85 résidus, une phase de lecture ouverte de 672 nucléotides (de la position 86 à 757) puis un 3' non traduit de 318 résidus.

La comparaison de la séquence protéique déduite de la séquence de l'ADNc avec celle obtenue par séquençage de la protéine native montre que l'ADNc code non seulement pour l'oxalate oxydase mature mais aussi pour un peptide signal de 23 acides aminés dans la partie N-terminale. L'oxalate oxydase est donc synthétisée sous forme d'une préprotéine (peptide signal plus peptide mature) qui est maturée par élimination du peptide signal pour libérer l'enzyme active mature.

Pour la suite nous utiliserons soit la partie codant pour la préprotéine (nucléotides 86 à 757), soit seulement la partie codant pour la protéine mature (de la position 155 à 757). Dans ce dernier cas, nous devrons placer un codon AUG (codant pour une méthionine) devant le codon ACC (codant pour la thréonine, premier acide aminé de la protéine mature).

Les attaques de plantes par Sclerotinia sclerotiorum se faisant essentiellement par la tige ou par la plante, il est intéressant de pouvoir exprimer l'oxalate oxydase soit dans les tissus chlorophylliens et pour cela on peut utiliser le promoteur de la petite sous-unité de la ribulose-1,5-bisphosphate carboxylase d'Helianthus annuus (PSUHa, Waksman et al., 1987) soit dans différents tissus du végétal et pour cela nous utiliserons le promoteur ubiquitaire de l'ARN 35S du virus de la mosaïque du chou-fleur (CaMV 35S) dont une partie a été dupliquée et qui est nommée "double CaMV"

Le document WO 88/08450 mentionne un gène chimère contenant une oxalate oxydase, mais ne mentionne ni ne suggère de l'exprimer dans les tissus végétaux pour conférer une résistance à Sclerotinia.

Les gènes chimères utilisables selon l'invention peuvent être par exemple construits à partir des éléments suivants:
A. Promoteur double CaMV suivi de la partie de l'ADNc de l'oxalate oxydase codant pour la préprotéine (petide signal plus peptide mature) et du terminateur "nos" obtenu à partir du gène de la nopaline synthase de pTi 37(Bevan et al., 1983).
B. Promoteur double CaMV suivi de la partie de l'ADNC de l'oxalate oxydase codant seulement pour la protéine mature suivi du terminateur "nos".
C. Gène identique à "A" mais avec le promoteur de la petite sous-unité de la ribulose-1,5-bisphosphate carboxylase de tournesol (PSUHa) à la place du double CaMV.
D. Gène identique à "B" mais avec le promoteur de la PSUHa à la place du double CaMV.

Chaque gène chimère est introduit dans la cellule végétale par un système utilisant Agrobacterium ou tout autre système connu par ailleurs pour transformer les cellules végétales. Des plantes sont régénérées à partir de ces cellules transformées. Elles présentent une tolérance accrue au Sclerotinia sclerotiorum.

### EXEMPLE 1 : Préparation de deux séquences codantes:

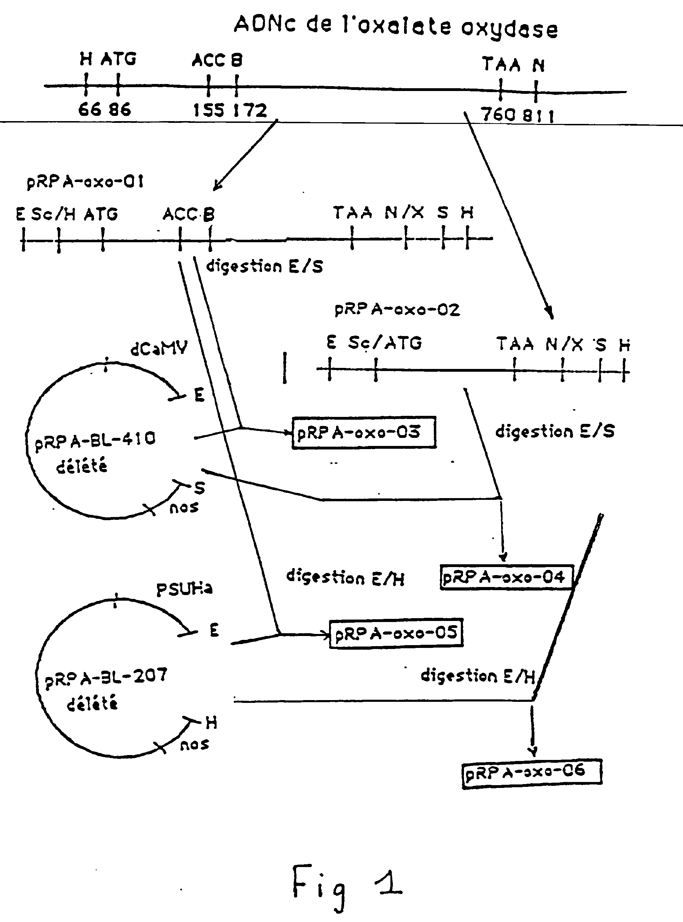

Préprotéine : on l'obtient à partir de l'ADNc décrit précédemment digéré par Hind III (en position 66). L'extrémité cohésive obtenue est rendue franche par traitement à la polymérase de Klenow. Cet ADN est ensuite digéré par Nhe I (en position 811).
   Parallèlement le plasmide pUC 19 (Yanisch-Perron et al., 1985) est digéré par Sac I.
L'extrémité cohésive obtenue est rendue franche par traitement à la polymérase de Klenow. Ensuite le Plasmide est digéré par Xba I (compatible avec Nhe I).
   Le fragment d'ADNc et le plasmide préparés ci-dessus sont ligaturés. Le nouveau plasmide ainsi obtenu est dénommé pRPA-oxo-O1 et sa carte est présentée figure 1.
B. Protéine mature : on l'obtient à partir de l'ADNc décrit ci-dessus après digestion par BstN I (en position 173). Le fragment obtenu et le linker ayant pour séquence : 
5'                    3'
ATGACCGACCCAGACCCTCTCC
 
TACTGGCTGGGTCTGGGAGAGGT
3'                    5'
 sont ligaturés. Ceci conduit à une modification de la séquence N-terminal de la protéine mature qui passe de TDPDPLQ à MTDPDPLQ.
   Ce fragment d'ADNc est ensuite digéré par Nhe I (en position 811) pour être ensuite ligaturé avec le plasmide pUC19 préparé comme décrit dans le paragraphe ci-dessus. Le nouveau plasmide ainsi constitué est appelé pRPA-oxo-02 et sa carte est présentée figure 1.

### EXEMPLE 2: Préparation des gènes chimères :

### a. Préparation des vecteurs comprenant le promoteur et le terminateur nos :

- exemple double CaMV : on obtient ce vecteur à partir du plasmide pRPA-BL-410 obtenu de la façon suivante:

### Fusion" Peptide de transit de la PSU de la RuBiscO de maïs/gène AroA":

Le peptide de transit de la PSU du gène de la RuBisCO de maïs provient d'un fragment EcoRI-SphI de 192pb;issu de l'ADNc correspondant au gène de la PSU du gène de la RuBisCO de maïs,décrit par Lebrun et al(1987), possédant un site NcoI recouvrant le codon initiateur de la traduction et un site SphI correspondant au site de clivage du peptide de transit.

Par traitement de l'extrémité SphI avec la polymerase du bactériophage T4 et en la ligaturant avec l'extrémité NcoI, traitée à la polymérase de Klenow, du gène AroA de pRPA-BL 104 recoupé EcoRI, on obtient la fusion traductionelle entre le peptide de transit de maïs et le gène de l'EPSPS bactérienne.

### Fusion peptide de transit de la PSU de la RuBisCO de maïs/séquence de 22 acides aminés de la partie mature de la PSU de la RuBisCO de maïs/gène AroA:

De façon similaire un fragment EcoRI-HindII de 228bp de l'ADNc de la PSU du gène de la RubisCO de maïs est ligaturé avec l'extrémité NcoI traitée à la polymerase de Klenow du gène AroA de pRPA-BL 104 et recoupé par EcoRI. On obtient une fusion traductionnelle entre le peptide de transit de la PSU de la RuBisCO de maïs,les 22 acides aminés de la partie mature de la PSU de la RuBisCO de maïs et le gène de l'EPSPS bactérienne.

### Peptide de transit de la PSU de la RuBisCO de tournesol:

Le fragment est issu de l'ADNc isolé par Waksman et Freyssinet(1987).Un site SphI a été créé selon la méthode de Zoller et Smith(1984 au site de clivage du peptide de transit.Le peptide de transit de la PSU de la RuBisCO de tournesol ainsi obtenu est un fragment EcoRI-SphI de 171 pb.

### Fusion peptide de transit de la PSU de la RuBisCO de tournesol/séquence de 22 acides aminés de la partie mature de la PSU de la RuBisCO de maïs/ gène AroA:

La construction contenant la fusion peptide de transit de la PSU de la RuBisCO de maïs/séquence de 22 acides aminés de la PSU de la RuBisCO de maïs de la partie mature du gène de maïs a été coupée par EcoRI-SphI de 171 pb correspondant au peptide de transit de la PSU dedu gène de la RuBisCO de tournesol. Une construction résultante présente une substitution des fragments EcoRI-SphI et est une fusion traductionnelle "peptide de transit de la PSU de la RuBisCO de tournesol/séquence de 22 acides aminés de la partie mature de la PSU de la RuBisCO de maïs/gène AroA.

Le fragment EcoRI-SalI a été ligaturé avec le fragment SalI-SstI contenantla séquence nos 3' et la bordure droite de l'ADN-T. Le fragment EcoRI-SstI résultant comprenant"peptide de transit de la PSU de la RuBisCO de tournesol/séquence de 22 acides aminés de la partie mature de la PSU de la RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV. La fusion transcriptionelle "double CaMV/peptide de transit de la PSU de la RuBisCO de tournesol/séquence de 22 acides aminés de la partie mature de la PSU de la RuBisCO de maïs/gène AroA/nos 3' dans le vecteur 150 A alpha 2 a été nommé pRPA-BL 294.

### Eusion"peptide de transit de la PSU de la RuBisCO de tournesol/séquence de 22 acides aminés de la PSU de la RuBisCO de maïs/peptide de transit de la PSU de la RuBisCO de maïs/gène AroA":

La construction ci-dessus est coupée par NcoI-HindIII libérant le gène AroA.Puis elle est mise en ligation avec un fragment NcoI-Hind III de 1,5kpb contenant la fusion"peptide de transit de la PSU de la RuBisCO de maïs/gène AroA". Une construction résultante présente une substitution des fragments NcoI-HindIII et est une fusion traductionelle "peptide de transit de la PSU de la RuBisCO de tournesol/séquence de 22 acides aminés de la PSU de la RuBisCO de la partie mature du gène de maïs /peptide de transit de la PSU de la RuBisCO de maïs/gène AroA".

Le fragment EcoRI-SalI a été ligaturé avec le fragment SalI-SstI contenantla séquence nos 3' et la bordure droite de l'ADN-T. Le fragment EcoRI-SstI résultant comprenant"peptide de transit de la PSU de la RuBisCO de tournesol/séquence de 22 acides aminés de la PSU de la RuBisCO de la partie mature du gène de maïs /peptide de transit de la PSU de la RuBisCO de maïs/gène AroA/nos 3'/Bordure droite ADN-T" est substitué au fragment EcoRI-SstI contenant la bordure droite de l'ADN-T du plasmide 150 A alpha 2 contenant le promoteur double CaMV.La fusion transcriptionelle "double CaMV/peptide de transit de la PSU de la RuBisCO de tournesol/séquence de 22 acides aminés de la PSU de la RuBisCO de la partie mature du gène de maïs/peptide de transit de la PSU de la RuBisCO de maïs/gène AroA/nos 3' dans le vecteur 150 A alpha 2 a été nommé pRPA-BL 410. Ce plasmide est digèré avec EcoRI et SalI pour enlever le gène de structure "peptide de transit optimisé-zone codant pour l'EPSPS mature", pRPA-BL-410 délété (voir figure 1).
- exemple PSUHa : on obtient ce vecteur à partir du plasmide pRPA-BL-207 (décrit dans la demande de brevet européenne O 337 899) que l'on digère avec Eco RI et Hind III pour enlever la zone codant pour la nitrilase, pRPA-EL-207 délété (voir figure 1).

### b. Construction des gènes chimères :

pRPA-oxo-O3 : il s'obtient en digérant pRPA-oxo-O1 par Eco RI et Sal I. Le fragment obtenu qui code pour la préprotéine est ensuite inséré entre les sites Eco RI et Sal I respectivement en aval du double CaMV et en amont du terminateur nos.

pRPA-oxo-04 : il s'obtient en digérant pRPA-oxo-02 par Eco RI et Sal I. Le fragment obtenu qui code pour la protéine mature est ensuite inséré entre les sites Eco RI et Sal I respectivement en aval du double CaMV et en amont du terminateur nos.

pRPA-oxo-05 : il s'obtient en digérant pRPA-oxo-01 par Eco RI et Hind III. Le fragment obtenu qui code pour la préprotéine est ensuite inséré entre les sites Eco RI et Hind III respectivement en aval du double PSUHa et en amont du terminateur nos.

pRPA-oxo-06 : il s'obtient en digérant pRPA-oxo-02 par Eco RI et Hind III. Le fragment obtenu qui code pour la protéine mature est ensuite inséré entre les sites Eco RI et Hind III respectivement en aval du promoteur PSUHa et du terminateur nos.

**Tableau 1 - Représentation schématique des quatre gènes chimères :**

| Identification | Promoteur | Zone codante oxalate oxydase | Terminateur |
|---|---|---|---|
| pRPA-oxo-03 | dCaMV | préprotéine | nos |
| pRPA-oxo-04 | dCaMV | mature | nos |
| pRPA-oxo-05 | PSUHa | préprotéine | nos |
| pRPA-oxo-06 | PSUHa | mature | nos |

### EXEMPLE 3: Obtention de colzas transgéniques:

### a. Transformation

Chaque vecteur tel que décrit ci-dessus est introduit dans la souche non-oncogène d'Agrobacterium tumefaciens EHA 101 (Hood et al., 1987) porteuse du cosmide pTVK 291 (Komari et al., 1986).

La technique de transformation du colza variété Westar suit essentiellement celle décrite par Boulter et al. (1990), en utilisant une concentration en bactéries de 2,5 x 10⁹ par ml (DO 600nm = 1).

### b. Régénération

La technique de régénération suit essentiellement celle décrite par Boulter et al. (1990). Les plantes sont enracinées sur le milieu de De Block et al. (1989). Elles sont ensuite conduite à floraison en serre.

### EXEMPLE 4: Mesure de la résistance du Colza à Sclerotinia sclerotiorum:

In vitro :
- Disques foliaires : la résistance est mesurée par pesée de la masse de trois disques foliaires au bout de 11 jours de croissance sur un milieu Murashige et Skoog(MS) avec hormones additionné de 1 mM d'acide oxalique.
   Dans ces conditions, on observe que pour les disques foliaires issus de Colzas modifiés avec l'un des gènes chimères pRPA-oxo-03, pRPA-oxo-04, pRPA-oxo-05 et pRPA-oxo-06, la masse des disques foliaires augmente nettement, alors que, pour les disques foliaires issus de colzas non modifiés, la masse stagne ou même diminue.
- Elongation racinaire : la résistance est aussi mesurée in vitro par mesure de l'élongation racinaire au bout de deux jours de croissance sur eau additionnée de 5 mM d'acide oxalique. On observe alors que les racines de plantes de Colzas modifiés, avec l'un des gènes chimères pRPA-oxo-03, pRPA-oxo-04, sont capables de pousser et de s'allonger, alors que les racines de Colzas non modifiés ne présentent, dans ces conditions, aucune croissance.

In vivo :
La résistance in vivo est mesurée en serre après contamination des plantes de Colza issues de la régénération, dès l'apparition des premières fleurs, soit par dépôt de spores de S. slerotiorum sur les pétales, l'infection des feuilles se faisant alors naturellement lors de la défloraison, soit par dépôt directement sur les feuilles de omycelium ou d'un pétale imprégné de mycelium. Les plantes modifiées par l'un des gènes chimères pRPA-oxo-03, pRPA-oxo-04, pRPA-oxo-05, et pRPA-oxo-06 ne laissent pas se développer le champignon et ne présentent aucun symptôme de pourriture caractéristique de la sclérotiniose, alors que des plantes non modifiées sont vite envahies de pourriture caractéristique du développement de Sclérotinia Sclerotiorum.

### Légende de la Figure :

Procédure d'obtention des quatres gènes chimères à partir des deux gènes de structure, pRPA-oxo-01 et pRPA-oxo-02 H, Hind III ; B, BstN ; N, Nhe I ; E, Eco RI ; Sc, Sac I ; S, Sal I et X, Xba I.

## Revendications

1. Procédé pour améliorer la résistance des plantes aux maladies fongiques, en particulier celles provoquées par *Sclerotinia sp.,* **caractérisé en ce que** l'on exprime dans lesdites plantes une protéine possédant une activité oxalate oxydase, ladite expression étant réalisée par transformation génétique desdites plantes avec un gène chimère comprenant une séquence codant pour une protéine possédant une activité oxalate oxydase.

2. Procédé selon la revendication 1, **caractérisé en ce que** ladite protéine est sous la forme d'une pré-protéine comprenant un peptide signal et un peptide mature, ledit peptide mature possédant une activité oxalate oxydase, provenant du même gène d'oxalate oxydase.

3. Procédé selon la revendication 2, **caractérisé en ce que** la pré-protéine est une germine.

4. Procédé selon la revendication 3, **caractérisé en ce que** la germine est une germine de blé.

5. Cellule végétale génétiquement modifiée, **caractérisée en ce qu'**elle contient un vecteur comprenant un gène chimère comprenant une séquence codant pour une protéine possédant une activité oxalate oxydase.

6. Cellule selon la revendication 5, **caractérisée en ce que** ladite protéine est sous la forme d'une pré-protéine comprenant un peptide signal et un peptide mature, ledit peptide mature possédant une activité oxalate oxydase, provenant du même gène d'oxalate oxydase.

7. Cellule selon la revendication 6, **caractérisée en ce que** la pré-protéine est une germine.

8. Cellule selon la revendication 7, **caractérisée en ce que** la germine est une germine de blé.

9. Cellule selon l'une des revendications 5 à 8, **caractérisée en ce que** la séquence codant pour la protéine possédant une activité oxalate oxydase est associée à un promoteur et un élément terminateur.

10. Cellule selon la revendication 9, **caractérisée en ce que** le promoteur est choisi parmi le promoteur de la petite sous-unité de la ribulose-1,5-bisphosphate carboxylase d'Helianthes anuus (PSUHa), et le promoteur dupliqué de l'ARN 35S du virus de la mosaïque du chou-fleur (double CaMV).

11. Cellule selon la revendication 9, **caractérisée en ce que** l'élément terminateur est la séquence nos.

12. Cellule selon l'une des revendications 5 à 11, **caractérisé en ce que** ledit gène chimère est choisi parmi les gènes chimères suivants :
a. gène chimère comprenant le promoteur double CaMV, la séquence codant pour la germine sous forme de pré-protéine, et le terminateur nos.
b. gène chimère comprenant le promoteur double CaMV, la séquence codant uniquement pour la germine sous forme de peptide mature, et le terminateur nos.
c. gène chimère comprenant le promoteur PSUHa, la séquence codant pour la germine sous forme de pré-protéine, et le terminateur nos.
d. gène chimère comprenant le promoteur PSUHa, la séquence codant uniquement pour la germine sous forme de peptide mature, et le terminateur nos.

13. Plante transformée issue d'une cellule selon l'une des revendications 5 à 12.

14. Plante selon la revendication 13, **caractérisée en ce qu'**elle est une dicotylédone.

15. Plante selon la revendication 14, **caractérisée en ce qu'**elle est du Colza.

## Claims

1. Method for improving the resistance of plants to fungal diseases, in particular those caused by *Sclerotinia sp.,* **characterized in that** the protein having oxalate oxidase activity is expressed in the said plants, the said expression being carried out by genetic transformation of the said plants with a chimeric gene comprising a sequence encoding a protein having oxalate oxidase activity.

2. Method according to Claim 1, **characterized in that** the said protein is in the form of a preprotein comprising a signal peptide and a mature peptide, the said mature peptide having oxalate oxidase activity derived from the same gene for oxalate oxidase.

3. Method according to Claim 2, **characterized in that** the preprotein is a germin.

4. Method according to Claim 3, **characterized in that** the germin is a wheat germin.

5. Genetically modified plant cell, **characterized in that** it contains a vector comprising a chimeric gene comprising a sequence encoding a protein having oxalate oxidase activity.

6. Cell according to Claim 5, **characterized in that** the said protein is in the form of a preprotein comprising a signal peptide and a mature peptide, the said mature peptide having oxalate oxidase activity derived from the same gene for oxalate oxidase.

7. Cell according to Claim 6, **characterized in that** the preprotein is a germin.

8. Cell according to Claim 7, **characterized in that** the germin is a wheat germin.

9. Cell according to one of Claims 5 to 8, **characterized in that** the sequence encoding the protein having oxalate oxidase activity is combined with a promoter and a terminator element.

10. Cell according to Claim 9, **characterized in that** the promoter is chosen from the promoter of the small subunit of ribulose-1,5-bisphosphate carboxylase of Helianthus annuus (SSUHa), and the duplicated promoter of the 35S RNA of the cauliflower mosaic virus (double CaMV).

11. Cell according to Claim 9, **characterized in that** the terminator element is the nos sequence.

12. Cell according to one of Claims 5 to 11, **characterized in that** the said chimeric gene is chosen from the following chimeric genes:
a. chimeric gene comprising the double CaMV promoter, the sequence encoding germin in preprotein form, and the terminator nos.
b. chimeric gene comprising the double CaMV promoter, the sequence encoding only germin in the form of a mature peptide, and the terminator nos.
c. chimeric gene comprising the SSUHa promoter, the sequence encoding germin in preprotein form, and the terminator nos.
d. chimeric gene comprising the SSUHa promoter, the sequence encoding only germin in the form of a mature peptide, and the terminator nos.

13. Transformed plant obtained from a cell according to one of Claims 5 to 12.

14. Plant according to Claim 13, **characterized in that** it is a dicotyledon.

15. Plant according to Claim 14, **characterized in that** it is the rape plant.

## Patentansprüche

1. Verfahren zur Verbesserung der Resistenz von Pflanzen gegenüber Pilzerkrankungen, insbesondere solchen, die von *Sclerotinia sp.* hervorgerufen werden, **dadurch gekennzeichnet, dass** in den Pflanzen ein Protein exprimiert wird, welches eine Oxalatoxidaseaktivität aufweist, wobei die Expression durch genetische Transformation der Pflanzen mit einem chimären Gen erzielt wird, das eine Sequenz umfasst, die für ein Protein kodiert, welches eine Oxalatoxidaseaktivität aufweist.

2. Verfahren nach Anspruch 1, **dadurch gekennzeichnet, dass** das Protein in Form eines Präproteins vorliegt, welches ein Signalpeptid und ein reifes Peptid umfasst, wobei das reife Peptid eine Oxalatoxidaseaktivität aufweist, die von demselben Oxalatoxidasegen herrührt.

3. Verfahren nach Anspruch 2, **dadurch gekennzeichnet, dass** es sich bei dem Präprotein um ein Germin handelt.

4. Verfahren nach Anspruch 3, **dadurch gekennzeichnet, dass** es sich bei dem Germin um ein Germin aus Weizen handelt.

5. Gentechnisch modifizierte Pflanzenzelle, **dadurch gekennzeichnet, dass** diese einen Vektor enthält, der ein chimäres Gen umfasst, welches eine Sequenz umfasst, die für ein Protein kodiert, welches eine Oxalatoxidaseaktivität aufweist.

6. Zelle nach Anspruch 5, **dadurch gekennzeichnet, dass** das Protein in Form eines Präproteins vorliegt, welches ein Signalpeptid und ein reifes Peptid umfasst, wobei das reife Peptid eine Oxalatoxidaseaktivität aufweist, die von demselben Oxalatoxidasegen herrührt.

7. Zelle nach Anspruch 6, **dadurch gekennzeichnet, dass** es sich bei dem Präprotein um ein Germin handelt.

8. Zelle nach Anspruch 7, **dadurch gekennzeichnet, dass** es sich bei dem Germin um ein Germin aus Weizen handelt.

9. Zelle nach einem der Ansprüche 5 bis 8, **dadurch gekennzeichnet, dass** die Sequenz, welche für das Protein kodiert, das eine Oxalatoxidaseaktivität aufweist, mit einem Promotor und einem Terminatorelement verbunden ist.

10. Zelle nach Anspruch 9, **dadurch gekennzeichnet, dass** der Promotor ausgewählt ist aus dem Promotor der kleinen Untereinheit der Ribulose-1,5-bisphosphatcarboxylase von Helianthus annuus (PSUHa) und dem duplizierten 35S RNA Promotor des Blumenkohlmosaikvirus (doppelter CaMV).

11. Zelle nach Anspruch 9, **dadurch gekennzeichnet, dass** es sich bei dem Terminatorelement um die Sequenz nos handelt.

12. Zelle nach einem der Ansprüche 5 bis 11, **dadurch gekennzeichnet, dass** das chimäre Gen ausgewählt ist aus den folgenden chimären Genen:
a. chimäres Gen, das den doppelten CaMV Promotor, die Sequenz, welche für Germin in Form des Präproteins kodiert, sowie den nos Terminator umfasst,
b. chimäres Gen, das den doppelten CaMV Promotor, die Sequenz, welche nur für Germin in Form des reifen Peptids kodiert, sowie den nos Terminator umfasst,
c. chimäres Gen, das den PSUHa Promotor, die Sequenz, welche für Germin in Form des Präproteins kodiert, sowie den nos Terminator umfasst,
d. chimäres Gen, das den PSUHa Promotor, die Sequenz, welche nur für Germin in Form des reifen Peptids kodiert, sowie den nos Terminator umfasst.

13. Transformierte Pflanze, die aus einer Zelle nach einem der Ansprüche 5 bis 12 hervorgeht.

14. Pflanze nach Anspruch 13, **dadurch gekennzeichnet, dass** es sich um eine Dicotyledone handelt.

15. Pflanze nach Anspruch 14, **dadurch gekennzeichnet, dass** es sich um Raps handelt.
